(19) Europäisches Patentamt
European Patent Office
Office européen des brevets

(11) Publication number: 0 185 823 B1

(12) EUROPEAN PATENT SPECIFICATION

(45) Date of publication of patent specification: 23.03.88

(51) Int. Cl.[4]: C 07 C 103/54, C 07 C 102/00, B 01 J 31/20

(21) Application number: 84309127.3

(22) Date of filing: 28.12.84

# (54) Dimethylacetamide from carbonylation of trimethylamine.

(43) Date of publication of application:
02.07.86 Bulletin 86/27

(45) Publication of the grant of the patent:
23.03.88 Bulletin 88/12

(84) Designated Contracting States:
BE DE FR GB IT NL

(56) References cited:
DE-C- 948 056
US-A-3 412 151

CHEMICAL ABSTRACTS, vol. 76, no. 9, 28 Feb 1972, Columbus, Ohio (US); N.ISOGAI: "N,N-Dialkylacylamides", p. 343, no. 45766q

CHEMICAL ABSTRACTS, vol. 91. no. 19, 5 Nov 1979, Columbus, Ohio (US); N.ISOGAI: "Manufacture of amides", p. 600, no. 157300z

(73) Proprietor: E.I. DU PONT DE NEMOURS AND COMPANY
1007 Market Street
Wilmington Delaware 19898 (US)

(72) Inventor: Bellis, Harold Edward
1 Hillvale Circle
Wilmington, DE 19808 (US)

(74) Representative: Barnard, Eric Edward et al
BROOKES & MARTIN High Holborn House
52/54 High Holborn
London WC1V 6SE (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Field of the Invention

The present invention relates to reacting trimethylamine with carbon monoxide to form dimethylacetamide.

Prior Art

U.S. Patent 3,412,151 discloses reacting trimethylamine with carbn monoxide to form dimethylformamide at elevated pressure and temperature in the presence an amount of water about equimolar to the trimethylamine reactant using dicobalt octacarbonyl as the catalyst.

Japanese Patent Publication No. 1971—43,526 discloses reacting trimethylamine with carbon monoxide to form dimethylacetamide using dicobalt carbonyl as the catalyst.

Summary of the Invention

The present invention relates to the discovery that when reacting trimethylamine with carbon monoxide using dicobalt octacarbonyl as the catalyst that the reaction can be effectively promoted with water as a hydrogen donor, while suppressing the formation of dimethylformamide by using from 0.5—2.0 moles of hydrogen donor per mole of dicobalt octacarbonyl.

Detailed Description

The present invention relates to the production of dimethylacetamide from trimethylamine and carbon monoxide. In accordance with the present invention from 0.5—2.0 and preferably 0.5—1.5 moles of a hydrogen donor per mole of $Co_2(CO)_8$ is present to form the active catalyst species. Thus, when using water as the hydrogen donor, the reaction is as follows:

$$Co_2(CO)_8 + H_2O + CO \rightarrow 2HCo(CO)_4 + CO_2$$

$$HCo(CO)_4 + (CH_3)_3N \rightarrow CH_3Co(CO)_4 + H(CH_3)_2N$$

$$CH_3Co(CO)_4 + CO \rightarrow CH_3\overset{\overset{\displaystyle O}{\|}}{C}Co(CO)_4$$

$$CH_3\overset{\overset{\displaystyle O}{\|}}{C}Co(CO)_4 + (CH_3)_3N \rightarrow CH_3\ \overset{\overset{\displaystyle O}{\|}}{C}N(CH_3)_3^+ + Co(CO)_4^-$$

$$\overset{\overset{\displaystyle O}{\|}}{CH_3}\ CN(CH_3)_3^+ + Co(CO)_4^- \rightarrow CH_3\overset{\overset{\displaystyle O}{\|}}{C}N(CH_3)_2 + CH_3Co(CO)_4$$

Thus $CH_3Co(CO)_4$ is the active catalyst species. This reaction has been found to depend on catalyst concentration and not CO concentration for its rate. Thus moderate pressures of as low as 400 psi ($2.7 \times 10^6$ pascals) can be used. While high pressures such as 4000 psi ($27.5 \times 10^6$ pascals) can be used, doing so is uneconomical. The preferred pressure range is from 600—2000 psi ($4.1 \times 10^6$ to $13.8 \times 10^6$ pascals). Generally the temperature used will be from 175—275°C with from 220—250°C being the preferred range.

Generally the process of the present invention produces less than 4% dimethylformamide and in its preferred mode less than 2% dimethylformamide. The product dimethylacetamide is widely used as a solvent and for most of these uses the presence of more than a few percent of dimethylformamide cannot be tolerated. Thus the process of the present invention provides a process for converting trimethylamine, which normally is a waste stream generated in the production of dimethylamine, into a useful product.

The process of the present invention can be performed as either a batch process or continuously.

The process can be carried out neat or using a solvent. Dimethylacetamide is the preferred solvent.

In a preferred aspect of the invention from 0.005—0.10 mole of a lower alkyl halide per mole of trimethyl amine is present in the reaction mixture. The preferred lower alkyl halide is methyl iodide used in a molar ratio of 0.5—2.0 moles of methyl iodide per mole of cobalt carbonyl.

Examples

Example 1

To a 200 ml reactor was charged 20 grams dimethylacetamide and 5 grams dicobalt octacarbonyl and 0.25 gram water in an atmosphere of nitrogen. The molar ratio of water to catalyst in this example was 0.95. The reactor was cooled to −80°C, evacuated and 50 grams trimethylamine was then added. The reactor was warmed to room temperature and pressured to 1000 psi ($6.9 \times 10^6$ Pa) with carbon monoxide. The vesel and contents were heated and maintained at 250°C for 6 hours. On reaching reaction temperature,

carbon monoxide was added to bring the pressure to 2500 psig (17.2 × $10^6$ Pa). After the pressure dropped to about 2300 psig (15.8 × $10^6$ Pa) it was repressured to 2500 psig (17.2 × $10^6$ Pa). The total amount of repressuring amounted to 2230 psig (15.4 × $10^6$ Pa). After 5 hours, CO absorption stopped. At the conclusion of the run, the reactor was cooled to room temperature and unreacted trimethylamine was vented into a cold trap maintained at −80°C. Unreacted trimethylamine amounted to 4.1 grams. The remaining product was then recovered and analyzed. The liquid product amounted to 74.9 grams. Dimethylacetamide amounted to 67.6 grams for a net conversion of trimethylamine to dimethylacetamide of 92%. Dimethylformamide represented 1% of the product. This example shows the beneficial effect of water.

Example 2 (comparative)

Example 1 was repeated but with 0.8 gram of water added to 5 grams dicobalt octacarbonyl. The molar ratio of water to catalyst in this example was 3.0. The reactor was also charged with 20 grams dimethylacetamide and 40 grams trimethylamine. The reactor was pressured to 1500 psig (10.3 × $10^6$ Pa) with carbon monoxide at room temperature. The reaction was run at 240°C and 3500 psig (24 × $10^6$ Pa) pressure for 2 hours. After the pressure dropped significantly the reaction was repressured to 3500 psig (24 × $10^6$ Pa). This was done repeatedly until the total amount of repressuring amounted to 4580 psig (31.6 × $10^6$ Pa). The product was recovered as before. Unreacted trimethylamine amounted to 5.1 grams. The liquid product amounted to 86.7 grams. Dimethylacetamide amounted to 71.5 grams for a net conversion of trimethylamine to dimethylacetamide of 85%. Dimethylformamide represented 9% of the product. This example shows the beneficial effect of a higher water/catalyst ratio on productivity but with more byproduct dimethylformamide.

Example 3 (comparative)

Example 1 was repeated but with only 0.07 gram water added to 5 grams dicobalt octacarbonyl. The molar ratio of water to catalyst in this example was 0.3. As before, the reactor was charged with 20 grams dimethylacetamide and 40 grams trimethylamine.

The reactor was pressured to 1500 psig (10.3 × $10^6$ Pa) with CO at room temperature. The reaction was run at 250°C and 3000 psig (24 × $10^6$ Pa) pressure for 8 hours. CO absorption was complete in 4 hours. After the pressure dropped significantly, the reactor was repressured to 300 psig (20.7 × $10^6$ Pa). This was done repeatedly until the total amount of repressuring amounted to 2860 psig (19.7 × $10^6$ Pa). The product was recovered as before. Unreacted trimethylamine amounted to 3.2 grams. The liquid product amounted to 69.4 grams. Dimethylacetamide amounted to 60.3 grams for a net conversion of trimethylamine to dimethylacetamide of 88%. Dimethylformamide represented 3% of the product. This example shows both lower productivity and higher byproduct formation at low water/catalyst ratios.

Example 4 (comparative)

Example 1 was repeated but with no water added to the 5 grams of dicobalt octacarbonyl. The reactor was charged with 20 grams dimethylacetamide and 40 grams trimethylamine. The reactor was pressured to 500 psig (3.45 × $10^6$ Pa) with CO at room temperature. The reaction was run at 250°C and 2000 psig (13.8 × $10^6$ Pa) pressure for 8 hours. After the pressure dropped significantly, the reactor was repressured to 2000 psig (18.3 × $10^6$ Pa). This was done repeatedly until the total amount of repressuring amounted to 1450 psig (10 × $10^6$ Pa) and was complete in 7 hours. The product was recovered as before. Unreacted trimethylamine amounted to 9.0 grams. The liquid product weighed 55.9 grams. Dimethylacetamide amounted to 44.6 grams for a net conversion of trimethylamine to dimethylacetamide of 93%. Dimethylformamide represented 3% of the product. This example shows low activity of the catalyst in the absence of water.

Exmaple 5

Example 1 was repeated with 0.3 gram of water added to 5 grams dicobalt octacarbonyl. The molar ratio of water/catalyst was 1.3. The reactor was charged with 20 grams dimethylacetamide and 40 grams trimethylamine. The reactor was pressured to 1500 psig (10.3 × $10^6$ Pa) with CO at room temperature. The reaction was run at 250°C and an initial CO pressure of 3800 psig (26.2 × $10^6$ Pa). There was no replenishment of CO. Rather, in this example the pressure was allowed to decrease as CO was absorbed. After 4 hours CO absorption was complete and the final pressure was 450 psig (3.1 × $10^6$ Pa). CO absorption totaled 3350 psig (23.1 × $10^6$ Pa). The product was recovered as before. Unreacted trimethylamine amounted to 1.5 grams. The liquid product totaled 76.9 grams. Dimethylacetamide amounted to 70.3 grams for a net conversion of trimethylamine to dimethylacetamide of 94%. Dimethylformamide represented 2% of the product. This example shows the beneficial effect of water on productivity and yield over a range of reactor pressure.

Example 6

In this example dimethylacetamide containing 50 g/gal (0.13 Kg/$m^2$) dicobalt octacarbonyl and 2 g/gal (0.005 Kg/$m^2$) water was fed to a 2 liter reactor at the rate of 2.4 liters/hour continuously over a 10 hour period. Trimethylamine was also fed at the rate of 1.3 lbs/hour (0.59 Kg/hour). The reactor was stirred and

maintained at 250°C and 1000 psig ($6.9 \times 10^6$ Pa) with carbon monoxide. Product was removed continuously through a low pressure separator. During this time, 5720 grams of trimethylamine was fed. Unreacted trimethylamine amounted to 2932 grams. The liquid product weighed 11,335 grams. Dimethylacetamide amounted to 10,219 grams for a net production of 2205 grams. Productivity was 0.73 lbs dimethylacetamide/gal/hour (53 $Kg/m^2/hr$). Conversion of trimethylamine to dimethylacetamide was 28%. The product contained 1.8% dimethylformamide. This example shows the beneficial effect of water for a continuous reaction.

Example 7

Example 1 was repeated but with some alkyl halide added with water to the catalyst. Methyl iodide (2.0 grams) was added to 0.5 gram water and 5.0 grams dicobalt octacarbonyl. The molar ratio of water/catalyst was 1.9. As before, the reactor was charged with 20 grams dimethylacetamide and 40 grams trimethylamine. The reactor was pressured to 2000 psig ($13.8 \times 10^6$ Pa) with carbon monoxide at room temperature. The reaction was run at 250°C and 5000 psig ($34.5 \times 10^6$ Pa) for 2 hours. CO absorption was complete after 68 minutes. After the pressure dropped significantly, the reactor was repressured to 2000 psig ($13.8 \times 10^6$ Pa). This was done repeatedly until the total amount of repressuring amounted to 5640 psig ($38.9 \times 10^6$ Pa). Unreacted trimethylamine amounted to 3.7 grams. Liquid product totaled 90.5 grams. Dimethylacetamide amounted to 81.4 grams and represents a 93% conversion of trimethylamine to dimethylacetamide. Dimethylformamide represented 4% of the product. This example shows the further improvement in reaction rate resulting from methyl iodide addition with water.

**Claims**

1. A process of producing dimethylacetamide containing less than 4% by weight dimethylformamide by intimately contacting a reaction mixture comprising trimethylamine, carbon monoxide in the presence of a catalytic amount of dicobalt octacarbonyl and water the molar ratio of water:dicobalt octacarbonyl being from 0.5—2.0:1 at a temperature of from about 175°C to about 275°C and a pressure of from $2.7 \times 10^6$ to $27.5 \times 10^6$ pascals (400—4000 psig).

2. The process of claim 1 wherein the reaction mixture further contains an alkyl halide is also added in a molar ratio of 0.005—0.10 alkyl halide to trimethylamine.

3. The process of claim 2 wherein the alkyl halide is methyl iodide.

4. The process of claim 3 wherein the methyl iodide is present in a molar ratio of 0.5—2.0 methyl iodide to cobalt carbonyl.

5. The process of claim 1 wherein the molar ratio of water:dicobalt octacarbonyl is from 0.5—1.5:1.

6. The process of claim 5 wherein the temperature is from 220—250°C.

7. The process of claim 6 wherein the pressure is $4.1 \times 10^6$ to $13.8 \times 10^6$ pascals (600—2000 psi).

**Patentansprüche**

1. Verfahren zur Herstellung von Dimethylacetamid, das weniger als 4 Gew.% Dimethylformamid enthält, durch inniges Inkontaktbringen von einer Trimethylamin enthaltenden Reaktionsmischung, Kohlenmonoxid in Gegenwart einer katalytischen Menge Dikobaltoctacarbonyl und Wasser, wobei das molare Verhältnis von Wasser:Dikobaltoctacarbonyl 0,5—2,0:1 beträgt, bei einer Temperatur von etwa 175°C bis etwa 275°C und einem Druck von $2{,}7 \times 10^6$ bis $27{,}5 \times 10^6$ Pascal (400 bis 4000 psig).

2. Verfahren nach Anspruch 1, worin die Reaktionsmischung weiterhin ein Alkylhalogenid in einem molaren Verhältnis von 0,005 bis 0,10 von Alkylhalogenid zu Trimethylamin zugesetzt enthält.

3. Verfahren nach Anspruch 2, worin das Alkylhalogenid Methyljodid ist.

4. Verfahren nach Anspruch 3, worin das Methyljodid in einem molaren Verhältnis von 0,5 bis 2,0 von Methyljodid zu Kobaltcarbonyl vorhanden ist.

5. Verfahren nach Anspruch 1, worin das molare Verhältnis von Wasser:Dikobaltoctacarbonyl 0,5—1,5:1 beträgt.

6. Verfahren nach Anspruch 5, worin die Temperatur 220 bis 250°C beträgt.

7. Verfahren nach Anspruch 6, worin der Druck $4{,}1 \times 10^6$ bis $13{,}8 \times 10^6$ Pascal (600 bis 2000 psi) beträgt.

**Revendications**

1. Un procédé de production de diméthylacétamide contenant moins de 4% en poids de diméthylformamide par mise en contact intime d'un mélange comprenant de la triméthylamine avec du monoxyde de carbone en présence d'une quantité catalytique de dicobalt-octacarbonyle et d'eau, le rapport molaire eau:dicobalt-octacarbonyle étant de 0,5:1 à 2,0:1, à une température d'environ 175°C à environ 275°C et sous une pression manométrique de $2{,}7 \times 10^6$ à $27{,}5 \times 10^6$ pascals.

2. Le procédé de la revendication 1, dans lequel le mélange réactionnel contient de plus un halogénure d'alkyle qui est ajouté en un rapport molaire de l'halogénure d'alkyle à la triméthylamine de 0,005—0,10.

3. Le procédé de la revendication 2, dans lequel l'halogénure d'alkyle est l'iodure de méthyle.

4. Le procédé de la revendication 3, dans lequel l'iodure de méthyle est présent en un rapport molaire de l'iodure de méthyle au cobalt-carbonyle de 0,5—2,0.

5. Le procédé de la revendication 1, dans lequel le rapport molaire eau:dicobalt-octacarbonyle est de 0,5:1 à 1,5:1.

6. Le procédé de la revendication 5, dans lequel la température est de 220 à 250°C.

7. Le procédé de la revendication 6, dans lequel la pression est de $4{,}1 \times 10^6$ à $13{,}8 \times 10^6$ pascals.